Europäisches Patentamt

(19) **European Patent Office**

**Office européen des brevets**

(11) Publication number: **0 059 882**
**A2**

(12) # EUROPEAN PATENT APPLICATION

(21) Application number: **82101384.4**

(22) Date of filing: **23.02.82**

(51) Int. Cl.³: **A 61 K 33/06**
**A 61 K 7/32**

(30) Priority: **23.02.81 US 236901**
**04.02.82 US 345368**

(43) Date of publication of application:
**15.09.82 Bulletin 82/37**

(84) Designated Contracting States:
**BE CH DE FR GB IT LI NL**

(71) Applicant: **COMBE INCORPORATED**
**1101 Westchester Avenue**
**White Plains New York 10604(US)**

(72) Inventor: **Lapidus, Herbert**
**46 Nutmeg Ridge**
**Ridgefield Connecticut 06877(US)**

(74) Representative: **Dost, Wolfgang, Dr.rer.nat.,**
**Dipl.-Chem. et al,**
**Patent- und Rechtsanwälte Pagenberg-Dost-Altenburg**
**Galileiplatz 1 Postfach 860 620**
**D-8000 Munchen 80 (86)(DE)**

(54) **Antipruritic composition.**

(57) An antipruritic composition comprising an anhidrotic and an emollient carrier as well as, optionally, a smoothening agent, a preservative bacteriacide and adjuvants.

EP 0 059 882 A2

Croydon Printing Company Ltd.

ANTIPRURITIC COMPOSITION

The present invention relates to antipruritic (anti-itch) compositions and to a method for treating bodily itches by using said antipruritic compositions. More particularly, the invention relates to the use of anhidrotics as the active ingredient in antipruritic compositions.

The physiological mechanism of the itching sensation is not presently known. Therefore, at least five different approaches have been used to relieve itching. Antimicrobial/antimitotic agents have been used to kill bacteria and reduce flaking skin (dandruff) and scalp itch. Anti-dandruff shampoos are perhaps the best examples of the use of these agents. Emollients such as vanishing creams, lotions, skin softeners and moisturizers have been used to smooth chapped, flaking and itching skin. Emollients are often found in water and oil emulsions. The water moistens the skin, and the oil prevents the water from leaving the skin, thereby soothing the skin and giving some relief. Organic substances, such as oatmeal, have been used for protectant, smoothing, and moisturizing purposes. Oatmeal apparently coats the skin while absorbing moisture. The moist oatmeal stays adjacent to the skin thereby keeping it moist and protecting it from other irritants. Astringents, such as Witch Hazel and alcohol, have also been used to reduce the itching sensation, although the reason for such relief is unknown. Finally, controlled substances or topical anesthetics such as ethylamino benzoate have been used to relieve itching. These substances, because of their physiological effects, anesthetize the sensory nerve endings in the vicinity of the itch and prevent the impulse from traveling to the receptor centers.

The inventor has not discovered the physiological itching mechanism, but has discovered that the anhidrotic aluminum chlorhydrate is extremely effective as the active ingredient of an antipruritic. Additionally, the inventor has discovered that anhidrotics, the active ingredients in antiperspirants, are effective as antipruritic agents as a class. Placing the anhidrotic in an emollient carrier to form an antipruritic cream, lotion, gel, ointment, suspension, dispersion, solution or powder vehicle ensures that the anhidrotic will remain in contact with the body when the antipruritic composition is placed on the body.

An antipruritic is provided comprising an anhidrotic and an emollient carrier. The anhidrotic is the active antipruritic ingredient with the emollient carrier ensuring that the anhidrotic remains in topical contact with the body. Aluminum chlorhydrate is an especially effective anhidrotic for these purposes. Smoothening agents, preservatives, and perfumes may all be added for cosmetic purposes. A method for relieving bodily itch is also provided and includes the step of placing a layer of cream, lotion, gel, ointment, suspension, dispersion, solution, or powder vehicle containing an anhidrotic in contact with the itching area of the body.

It is, therefore, an object of this invention to provide an antipruritic composition which may be used to eliminate itching.

Another object of this invention is to provide antipruritic compositions which are as effective as topical anesthetic antipruritics, but which contain anhidrotics used in common antiperspirants.

These, and other objects and advantages of the invention will become more apparent from the detailed description and the appended claims.

## PREFERRED EMBODIMENT OF THE INVENTION

The present invention comprises an antipruritic composition and a method for relieving a bodily itch by using such antipruritic composition, wherein an anhidrotic is the active ingredient in the antiprurient. An emollient carrier is added to the anhidrotic to form an antiprurient composition and the emollient carrier insures that the anhidrotic remains in contact with the body when the antipruritic composition is placed on the body. Thickening or smoothening agents, perfumes, and bacteriacides or preservatives may be added for cosmetic purposes.

It has been discovered that anhidrotics as a class, and in particular aluminum chlorhydrate are most effective antiprurients, and when placed in an emollient carrier, such as an oil in water vanishing cream, effectively relieve itching. It should be understood, of course, that the anhidrotics are the active ingredients of the antipruritic. The amount of anhidrotic employed in the composition may vary generally within conventional limits. Customarily, however, the anhidrotic is present in an amount up to 50 percent of the total weight of the composition it is in.

As aforementioned, it is believed that anhidrotics as a class are effective as the active ingredients in antipruritic compositions. Members of that class, including aluminum chlorhydrate, aluminum chloride, aluminum sulfate and aluminum/ zirconium trichlorohydrate have proven so effective. Thus, the following non-exhaustive list of anhidrotic chemicals are believed useful as antipruritics. These chemicals are active water-soluable ionizing antiperspirant chemicals capable of yielding antiperspirant active ion species. Preferably, the active ion species contain aluminum, zirconium or zinc. These chemicals are as follows:

| Aluminum Chloride | Zirconium Chloride |
| Aluminum Hydroxy Chloride (chlorydrate) | Zirconium Hydroxy Chloride |
| Aluminum Formate | Zirconium Lactate |
| Aluminum Sulfamate | Zirconium Oxy Chloride |
| Aluminum Phenol Sulfate | Zirconium Tartrate |
| Aluminum Phenol Sulfonate | Zirconium Carbonate |
| Aluminum Phosphite | |
| Aluminum Sulfate | Aluminum Zirconium Tetrachlorohydrate |
| Aluminum Methionate | Aluminum Zirconium Trichlorohydrate |
| Aluminum Acetate | |
| Aluminum Allantoinate | Zinc Methionate |
| Aluminum Citrate | Zinc Chloride |
| Aluminum Glutamate | Zinc Sulfate |
| Aluminum Sulfocarbolate | |

Similarly, other anhidrotics, such as are listed in an article entitled "Recent Advances in Antiperspirant Research," by B.B. Michniak, in Cosmetic Technology, Nov. 1980, p.36, should be effective as antiprurients. While, anhidrotics as a class are effective as antiprurients, care should be exercised, as some of them may not be used due to their toxicity, tendancy to discolor, or implication in the development of skin granulomas, as is well known in the chemical antiperspirant arts.

According to the invention, all that is needed to make an antiprurient composition is an anhidrotic and an emollient carrier. As many anhidrotics are available to satisfy the invention, so many emollient carriers are also available. Any emollient carrier or equivalent that will act to keep the anhidrotic in contact with the itching body will suffice. Typically, the emollient carrier will contain an emulsifier which will permit the dispersement of the water and oil in an emulsion form. However, the selection of the emollient is purely a matter of taste. In fact, the form of the carrier for the antiprurient can be chosen as desired and while not limited thereto, may be a vanishing cream, lotion, gel, ointment solution, suspension, dispersion, or powder, which are accomplished by means well known in the cosmetic arts. It is desirable, however, to maintain a proper pH level so that dermal irritation will not result from the application of the antipruritic composition to the skin.

The use of perfume is also purely a matter of individual choice. Likewise, the use of an antibacterial preservative is not mandatory, but might be desirable to prevent bacterial growth in said composition, which can be introduced by the user of the antipruritic composition. Supplementary emulsifiers and emollients may be added as wished. Smoothening or protecting agents for the skin may be added for texture purposes or for supplementary skin protection. Finally, adjuvants may be added to the anhidrotics, and is believed that astringents might be helpful in this regard.

It was desired to obtain an antiprurient with a desirable cosmetic texture and aroma, so 33.4 kg of deionized water was placed in a suitable container and 2.5 kg of a smoothening agent, colloidal oat flour such as Ster-O-Pro® produced by the Quaker Oats Co., was dispersed therein. The oat flour-water mixture was heated to 65° while being mixed, and 3 Kg of 50 percent solution aluminum chlorhydrate, an anhidrotic, was added. In a separate stainless steel container, 5 kg of Promulgen D®, an emulsifier produced by Robinson-Wagner Co., containing cetearyl alcohol and ceteareth-20®, 2.6 kg of the emulsifier cetyl alcohol, and 2.5 kg of Emollient 60®, produced by Inolex, containing isopropyl palmitate, isopropyl myristate and isopropyl stearate, were mixed together and brought to 60°C. The contents of the stainless steel container were than added to the oat flour-water-aluminum chlorhydrate container with mixing. The mixture was then cooled to 50°C and 0.025 kg the preservative-bacteriacide Zinc Omadine® produced by Olin Corp., was added with mixing. The contents were then cooled to 40°C and 1 kg of the adjuvant astringent SD40 Alcohol® (ethanol) produced by U.S.I., and 0.05 kg of perfume were added with mixing. The contents were cooled to 30°C with continued stirring and then homogenized to form a cosmetically suitable antipruritic composition.

A layer of the antipruritic composition containing the anhidrotic aluminum chlorhydrate was placed on the itching portion of a body and found to be at least as effective in relieving the itch as a commercially available antipruritic containing ethylamino benzoate.

Similarly, aluminum chloride or aluminum sulfate were used in the composition as described above to replace the aluminum chlorhydrate and produced antipruritic compositions that were effective in relieving itches when placed in contact with an itching portion of a body.

The following numbered antipruritics also illustrate other representative formulations embodying the discovery of the present invention.

<u>Anti-Itch Lotion # 1</u>

1. Polyoxyproplyene Fatty Alchol Ethers, E-SP     5.00%
   (Arlamol E-SP)® (I.C.I.)

2. Polyoxyethylene (2) Stearyl Ether (Brij 72)®   2.00
   (I.C.I.)

3. Polyoxyethylene (20) Stearyl Ether (Brij 78)®  1.50
   (I.C.I.)

4. DiSodium Edetate, DiHydrate                    0.10

*5. Aluminum Chlorhydroxide (50% soln.)           16.00

*6. Aluminum Chloride 6H$_2$0 (50% soln.)          8.00

7. Water                                          67.40
                                                 100.00%

<u>Anti-Itch Cream #2</u>

1. Propylene Glycol                               5.00%

2. Promulgen "G"®(Robinson-Wagner Co.)            4.00
   Emulsifier

3. Glyceryl Monostearate                          7.00

4. Isopropyl Myristate                            2.00

*5. Rezal 36 G.® (Reheis Chemical)(36% Active)    50.00
    Aluminum/Zirconium Trichlorohydrate

6. Titanium Oxide                                 0.50

7. Water                                          31.50
                                                 100.00%

* Active antiprurient ingredients

Layers of Anti-Itch Lotion #1 and Anti-Itch Cream #2 were placed on itching portions of a body and were found to be effective in relieving itches. Thus, it is seen that various anhidrotics in various concentrations prove effective as antipruritics. Aluminum Chlorhydrate 50% solution, having been 6% of the total weight of the antipruritic composition, and thus 3% by weight out of solution, was effective. Likewise, in Anti-Itch Cream #2, the Aluminum/Zirconium trichlorohydrate was effective while constituting 50% by weight of the composition.

The invention thus makes use of the discovery that anhidrotics are very effective as the active ingredients in antipruritic compositions.

Further, a method for relieving an itching body which utilizes said aforementioned antipruritic compositions is provided.

The following part of the description are preferred embodiments 1 to 21 presented in the form of claims.

1. An antipruritic composition comprising: an anhidrotic, and an emollient carrier.

2. An antipruritic composition comprising: an anhidrotic, and an emollient carrier, but excluding the making using or selling of said composition for all purposes except as an antipruritic composition.

3. An antipruritic composition as claimed in Claim 2 further comprising: a smoothening agent.

4. An antipruritic composition as claimed in Claims 2 or 3 wherein: said anhidrotic is aluminum chlorhydrate.

5. An antipruritic composition as claimed in Claim 4 wherein: said emollient carrier contains at least one oil, water, and at least one emulsifier.

6. An antipruritic composition as claimed in Claim 5 wherein: said smoothening agent is oat flour.

7. An antipruritic composition as claimed in Claim 6 wherein: said emollient carrier is selected from the class of vanishing cream, lotion, gel, ointment, suspension, dispersion, solution or powder.

8. An antipruritic composition as claimed in Claim 7 further comprising a preservative bacteriacide.

9. An antipruritic composition as claimed in Claim 8 wherein: said preservative bacteriacide is zinc pyrithione.

10. An antipruritic composition as claimed in Claim 9 wherein: said emulsifiers include cetearyl alcohol and ceteareth-20, and cetyl alcohol; said oils include isopropyl palmitate, isopropyl myristate and isopropyl stearate; and said water is deionized water.

11. An antipruritic composition as claimed in Claim 10 further comprising perfume.

12. An antipruritic composition as claimed in Claim 11 further comprising adjuvants.

13. An antipruritic composition as claimed in Claim 12 wherein; said adjuvants are astringents.

14. The antipruritic composition of Claims 2 or 3 wherein: the total weight of said anhidrotic out of solution is less than or equal to 50% of the total weight of said antipruritic composition.

15. The antipruritic composition of Claim 1 wherein: the weight of the said anhidrotic out of solution is greater than 2% of the total weight of said antipruritic composition.

16. The antipruritic composition of claim 15 wherein said anhidrotic is aluminum chlorhydrate.

17. The antipruritic composition of Claim 16 wherein said emollient carrier contains at least one oil, water, and at least one emulsifier and wherein said smoothening agent is oat flour.

18. An antipruritic composition as claimed in Claim 17 wherein: said emollient carrier is selected from the class of vanishing cream, lotion, gel, ointment, suspension, dispersion, solution or powder.

19. A method of treating an itching part of a body comprising: placing a layer of antipruritic creams on said itching part of said body, said antipruritic containing an anhidrotic which acts as the active ingredient in said antipruritic, and an emollient carrier.

20. A method of treating an itching part of a body as in Claim 19 wherein said antipruritic further contains a smoothening agent.

21. A method of treating an itching part of a body as in Claim 19 or 20 wherein: said anhidrotic is aluminum chlorhydrate.

PATENT- UND RECHTSANWÄLTE

RECHTSANWALT

JOCHEN PAGENBERG DR JUR. LL M HARVARD

PATENTANWÄLTE·

WOLFGANG A. DOST DR . DIPL .CHEM ·
UDO W. ALTENBURG DIPL .PHYS·

POSTFACH 86 06 20, 8000 MÜNCHEN 86
GALILEIPLATZ 1, 8000 MÜNCHEN 80
TELEFON (0 89) 98 03 61
TELEX (05) 22 791 pad d
CABLE· PADBÜRO MÜNCHEN

DATUM February 23, 1982
B 2377 D/ra

**0059882**

## C l a i m s

1. An antipruritic composition comprising an anhidrotic and an emollient carrier as well as, optionally, a smoothening agent, a preservative bacteriacide and adjuvants.

2. A composition according to claim 1 characterized in that said anhidrotic is aluminum chlorhydrate.

3. A composition according to claim 1 or 2 characterized in that said emollient carrier contains at least one oil, water, and at least one emulsifier.

4. A composition according to at least one of claims 1 to 3 characterized in that said smoothening agent is oat flour.

5. A composition according to at least one of claims 1 to 4, characterized in that said emollient carrier is selected from vanishing cream, lotion, gel, ointment, suspension, dispersion, solution or powder.